# EUROPEAN PATENT APPLICATION

(11) **EP 3 812 010 A1**
(43) Date of publication of application: **28.04.2021**
(21) Application number: 19205504.4
(22) Date of filing: 25.10.2019
(51) Int. Cl.: A61Q 5/00, A61Q 5/12, A61K 8/41, A61K 8/362, A61K 8/365

(54) **HAIR CONDITIONING COMPOSITIONS**

(71) Applicant: Unilever PLC, London Greater London EC4Y 0DY (GB)
(72) Inventor: LEE, Kenneth Stuart., Bebington, Wirral Merseyside CH63 3JW (GB); PAUL, Prem Kumar Cheyalazhagan., Spital, Wirral Merseyside CH63 9LE (GB)
(74) Representative: Chisem, Janet

(57) **Abstract**

A conditioner composition comprising
(i) an organic acid selected from butane tetra carboxylic acid, fumaric acid, tartaric acid and combinations thereof; and
(ii) a cationic conditioning surfactant;
wherein the composition has a pH of from 3 to 6.5, provides repair to damaged internal protein of hair.

## Description

### FIELD OF THE INVENTION

The invention relates to the use of hair treatment compositions in the repair of hair damage.

### BACKGROUND AND PRIOR ART

Consumers regularly subject their hair to intensive treatment, and care and styling routines to help them achieve their desired look. The actions performed by consumers introduce modifications to the chemistry of hair keratin protein which results in micro- and macro-structural changes and, in turn, changes fibre physical properties: the consequences of these are generally perceived by the consumer as damage.

Combing and brushing of hair mechanically abrades the fibre cuticle making this rougher and increasing the frictional characteristics. Hair lightening, such as bleaching, or colouring treatments generally involve an oxidative step to break down melanin and develop the new hair colour, but these processes also oxidise the hair fibre protein and the endogenous lipids. These reactions alter the number and types of covalent and noncovalent bonds within the fibre and impact the thermal stability and mechanical properties of the hair. The internal protein of damaged hair typically has a reduced denaturation temperature compared to that of virgin hair.

Various organic acids, organic molecules and combinations thereof have been suggested for use in the treatment of damaged hair.

WO 2004054526 describes hair treatment compositions for the care and repair of damaged hair, and for improving hair manageability, comprising a disaccharide and a di-acid, especially succinic acid and adipic acid.

WO2005/094761 discloses a combination of a disaccharide, a di-acid, particularly succinic acid and adipic acid and a source of ammonium ions, preferably ammonium carbonate to deliver styling, smoothing, alignment and damage prevention benefits to hair.

WO2005/097048 discloses a hair treatment composition such as a shampoo or conditioner comprising a disaccharide, alkali metal salt and a di-acid or salt thereof. Use of said composition for smoothing and aligning hair, for preventing damage to the hair is disclosed.

WO2016/188691, WO2017/157993 and WO2019/030034 disclose the use of a hair treatment composition comprising a lactone, a disaccharide, an inorganic salt and an organic acid or salt thereof, having a pH in the range of from 3 to 6.5, in the treatment of hair, to repair damage to hair protein. Suitable acids include glycolic acid, lactic acid, citric acid, mandelic acid, propanoic acid, betahydroxypropionic acid, betahydroxybutyric acid, salicylic acid, carnitine and mixtures thereof.

### DEFINITION OF THE INVENTION

In a first aspect of the invention, there is provided a conditioner composition comprising
(i) an organic acid selected from butane tetra carboxylic acid, fumaric acid, tartaric acid and combinations thereof; and
(ii) a cationic conditioning surfactant;
wherein the composition has a pH of from 3 to 6.5.

The present invention provides damage repair to hair, preferably an increase in the denaturation temperature of the internal protein of hair.

A second aspect of the invention provides a method of treating hair, comprising the step of applying a conditioner composition of the first aspect.

Preferably, the method provides a step of rinsing the composition from the hair.

A use is also provided, of a composition comprising an organic acid selected from butane tetra carboxylic acid, fumaric acid, tartaric acid and combinations thereof. and a cationic conditioning surfactant to repair damage to hair protein.

### The Hair

The hair is preferably damaged hair, where the denaturation temperature of the internal protein of the hair is reduced.

The damage may be caused by mechanical means, for example combing and brushing, chemical means, exposure to heat, environmental means such as sunlight and exposure to damaging energy sources, for example light such as UV light. Chemical means includes treatments that involve an oxidative step, for example, hair lightening, such as bleaching, and colouring treatments. Preferably the hair is bleached, more preferably bleached multiple times.

### GENERAL DESCRIPTION OF THE INVENTION

### Conditioners

The composition of the invention has a pH of from 3 to 6.5, preferably 3.5 to 5.

Conditioner compositions will typically comprise one or more cationic conditioning surfactants which are cosmetically acceptable and suitable for topical application to the hair.

Preferably, the cationic conditioning surfactants have the formula N⁺(R¹)(R²)(R³)(R⁴), wherein R¹, R², R³ and R⁴ are independently (C₁ to C₃₀) alkyl or benzyl.

Preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic conditioning surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic conditioning surfactants for use in the invention, either alone or in admixture with one or more other cationic conditioning surfactants, is a combination of (i) and (ii) below:
(i) an amidoamine corresponding to the general formula (I): 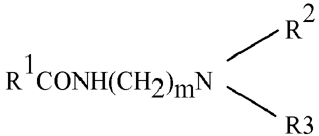
   in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.
As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and
m is an integer from 1 to about 4.

Preferably, R² and R³ are methyl or ethyl groups.

Preferably, m is 2 or 3, i.e. an ethylene or propylene group.
Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachid-amidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudoquaternary ammonium cationic surfactant.

Suitably, the acid is included in a sufficient amount to protonate all the amidoamine present, i.e. at a level which is at least equimolar to the amount of amidoamine present in the composition.

In conditioners of the invention, the level of cationic conditioning surfactant will generally range from 0.01 to 10%, more preferably 0.05 to 7.5%, most preferably 0.1 to 5% by total weight of cationic conditioning surfactant based on the total weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

### Acids:

The compositions of the present invention comprise an organic acid selected from butane tetra carboxylic acid, fumaric acid, tartaric acid and combinations thereof. Preferably the organic acid is tartaric acid. Preferably the amount of organic acid is in the range of from 0.01 to 5 wt. %, by weight of the total composition.

### Form of Composition

Hair treatment compositions according to the invention, particularly water- hair conditioners, will preferably also contain one or more silicone conditioning agents.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula:

HO(CH₂CH₂O)ₓ(CH(CH₃)CH₂O)_{y}(CH₂CH₂O)ₓ H

wherein the mean value of x is 4 or more and the mean value of y is 25 or more.

Another preferred form of the surface active block copolymer is according to the following formula:

(HO(CH₂CH₂O)ₐ(CH(CH₃)CH₂O)_{b})₂-N-CH₂-CH₂-N((OCH₂CH(CH₃))_{b}(OCH₂CH₂)ₐOH)₂

wherein the mean value of a is 2 or more and the mean value of b is 6 or more.

Mixtures of any of the above described silicone emulsions may also be used.
The above described silicone emulsions will generally be present in a composition of the invention at levels of from 0.05 to 10%, preferably 0.05 to 5%, more preferably from 0.5 to 2% by total weight of silicone based on the total weight of the composition.

### Other Ingredients

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally, these optional ingredients are included individually at a level of up to 5% by weight of the total composition.

Hair treatment compositions of the invention are primarily intended for topical application to the hair and/or scalp of a human subject, either in rinse-off or leave-on compositions, for the treatment of dry, damaged and/or unmanageable hair.

The invention will be further illustrated by the following, non-limiting Example, in which all percentages quoted are by weight based on total weight unless otherwise stated.

### EXAMPLES

### The Hair

Virgin: The hair used in the following examples was dark brown European hair tresses 2.5 grams and 6 inches long.

Bleached: Virgin hair tresses were bleached according to the following protocol. Hair was bleached for 30 minutes with commercial bleaching powder mixed with 9% cream peroxide, 30 'vol'. Hair was then washed with 14% aqueous sodium laureth ether sulphate (SLES) solution before drying.

The bleached hair was dialysed prior to the experiments in 5L of distilled water over a period of 72 hours, and the water was changed 3 times over this period. After dialysing, the hair tresses were left to dry overnight in a controlled environment (20°C and 50% relative humidity).

Hair damage repair can be measured by the denaturation temperature of the hair fibre. Without wishing to be bound by theory, it is believed that some acids can penetrate the hair fibre and help to re-build damaged protein structures. This in turn leads to the increased denaturation temperature of the fibre. Therefore, higher denaturation temperature indicates damage repair in a fibre.

### Denaturation temperature (Td) Model:

A reliable Td model has been used to investigate which acids provide damage repair to hair fibres via one shampoo wash at pH 4.5 (i.e. which acids increase the denaturation temperature of the hair fibre). The model calculates Td values for acids based on: % level of acid, pKa of the acid, molecular weight, number of rotatable bonds, number of hydrogen bond acceptors, number of hydrogen bond donners, partition coefficient Alog P, solubility measured as log S and Apol.

The model has been validated against measured Td values.

**Table 1 - Comparison of Predicted Td for various acids at 1 wt.%:**

| | Predicted Td |
|---|---|
| Tartronic acid | 144.8 |
| Gluconic acid | 146.8 |
| Itaconic acid | 148.0 |
| Acetic acid | 148.2 |
| Malonic | 148.25 |
| Malic | 148.25 |
| Butane Tetra Carboxylic acid | 148.8 |
| Fumaric acid | 149.2 |
| Tartaric acid | 149.6 |

### Untreated hair has a Td of: 148.0

Using the denaturation temperature model, treatment with butane tatra carboxylic acid, fumaric acid and tartaric acid are identified as providing the highest denaturation temperature, i.e. the best damage repair.

**Example formulation:**

| **Ingredient** | **Amount (wt. %)** |
|---|---|
| Acid¹ | 1 |
| Cetearyl Alcohol | 4 |
| Behentrimonium Chloride | 1 |
| Stearamidopropyl Dimethylamine | 1 |
| Silicone | 2.14 |
| Sodium Chloride | 0.1 |
| Perfume | 0.65 |
| Water & minors | to 100 |
| pH | 3.9 |

| | |
|---|---|
| Acid¹ - in accordance with the present invention | |

## Claims

1. A conditioner composition comprising
(i) an organic acid selected from butane tetra carboxylic acid, fumaric acid, tartaric acid and combinations thereof; and
(ii) a cationic conditioning surfactant;
wherein the composition has a pH of from 3 to 6.5.

2. The composition as claimed in claim 1, wherein the organic acid is tartaric acid.

3. The composition as claimed in any preceding claim, wherein the amount of organic acid is in the range of from 0.01 to 5 wt %, by weight of the total composition.

4. The composition as claimed in any preceding claim, wherein the cationic conditioning surfactant is selected from cetyltrimethylammonium chloride and behenyltrimethylammonium chloride.

5. The composition as claimed in any preceding claim, wherein the pH is from 3.5 to 5.

6. A method of treating hair, comprising the step of applying a composition as claimed in any preceding claim, to hair.

7. A method as claimed in claim 6, wherein the hair is bleached.

8. Use of a composition comprising an organic acid selected from butane tetra carboxylic acid, fumaric acid, tartaric acid and combinations thereof, to repair damage to hair protein.

9. Use as claimed in claim 8, wherein the composition is a conditioner composition, further comprising a cationic conditioning surfactant.

## Amended claims

### Amended claims in accordance with Rule 137(2) EPC.

1. A conditioner composition comprising
(i) 0.01 to 5 wt % of an organic acid selected from butane tetra carboxylic acid, a combination of butane tetra carboxylic acid with fumaric acid and a combination of butane tetra carboxylic acid with tartaric acid;;
(ii) 0.1 to 10 wt % of a cationic conditioning surfactant; and
(iii) a silicone emulsion;
wherein the composition has a pH of from 3 to 6.5.

2. The composition as claimed in claim 1, wherein the organic acid is butane tetra carboxylic acid.

3. The composition as claimed in any preceding claim, wherein the amount of cationic conditioning surfactant is in the range of from 0.1 to 5 wt %, by weight of the total composition.

4. The composition as claimed in any preceding claim, wherein the cationic conditioning surfactant is selected from cetyltrimethylammonium chloride and behenyltrimethylammonium chloride.

5. The composition as claimed in any preceding claim, wherein the pH is from 3.5 to 5.

6. The composition as claimed in any preceding claim, wherein the silicone emulsion is present in an amount of from 0.5 to 2 wt %.

7. A method of treating hair, comprising the step of applying a composition as claimed in any preceding claim, to hair.

8. A method as claimed in claim 6, wherein the hair is bleached hair.

9. Use of a composition as defined in any of claims 1 to 6, to repair damage to hair protein.
